# EUROPEAN PATENT SPECIFICATION

(11) **EP 1 251 875 B1**
(45) Date of publication and mention of the grant of the patent: **15.03.2006**
(21) Application number: 00993689.9
(22) Date of filing: 21.12.2000
(51) Int. Cl.: A61K 49/00

(54) **USE OF CONTRAST AGENTS IN THE MANUFACTURE OF DIAGNOSTIC AGENTS FOR THE VISUALISATION OF THE INTESTINAL LUMEN**
VERWENDUNG VON KONTRASTMITTELN ZUR HERSTELLUNG EINES DIAGNOSTISCHEN MITTELS FÜR DIE DARM-LUMENBILDGEBUNG
UTILISATION D'AGENTS DE CONTRASTE DANS LA FABRICATION D'AGENTS DIAGNOSTIQUES POUR LA VISUALISATION DE LA LUMIERE INTESTINALE

(30) Priority: 29.12.1999 IT MI992735; 08.02.2000 IT MI000193
(43) Date of publication of application: 30.10.2002
(73) Proprietor: BRACCO IMAGING S.p.A., 20134 Milano (IT)
(72) Inventor: KNOPP, Michael V., 69207 Sandhausen (DE); GIESEL, Frederik, 69117 Heidelberg (DE); VON TENGG-KOBLIGK, Hendrik, 48155 Münster (DE); RADELEFF, Jannis, 69121 Heidelberg (DE)
(74) Representative: Minoja, Fabrizio
(86) International application number: PCT/EP2000/013069
(87) International publication number: WO 2001/049326

(56) References cited:
- WO-A-93/03351
- WO-A-93/10821
- WO-A-93/15093
- US-A- 5 401 493

## Description

This invention relates to the use of particular contrast agents to prepare diagnostic compounds suitable for non-invasive visualisation under physiological conditions of the intestinal lumen, and the colon in particular.

The importance of acquiring diagnostically useful images of the colon can be deduced from the importance of the diseases affecting the colon. For example, cancer of the colon is one of the most frequent malignant diseases among the male population.

To date, the diagnostic technique most frequently used for gastrointestinal imaging, especially of the colon, has been X-ray radiography performed after oral or aboral administration of barium sulphate, suitably formulated in suspension. The main drawback of this technique, however, is its frequently low level of diagnostic usefulness caused by uneven distribution of the contrast medium in the intestine. This can occur for various reasons, for example, presence of faeces, diverticuli and so on. Furthermore, ingestion of the product often causes great discomfort for patients.

Magnetic resonance imaging (MRI) colonography is a recently introduced technique that allows certain evaluation of colon diseases, in which the diagnostic images are preferably acquired by means of three-dimensional imaging of transverse sections of the regions concerned. However, this diagnostic technique requires oral or rectal administration of exogenous contrast agents, and frequently requires simultaneous intravenous administration of a suitable MRI contrast agent to visualise the vascular system.

Oral or aboral administration of contrast agents usually requires adequate prior preparation of the patient. This preparation involves emptying as completely as possible the intestinal tract to be subjected to diagnostic investigation, and distending it by further administration of suitable relaxant substances. Images of the intestine or colon can obviously not be obtained in this way under physiological conditions. In this case too, the patient suffers considerable discomfort.

Another recently introduced technique is computerised tomography (CT) colonography; however, this technique also requires oral or rectal administration of a suitable contrast agent and adequate preparation of the patient.

Basically, each of the techniques described is more or less invasive, and above all involves discomfort for the patient. Moreover, none of them allows the examination to be conducted under physiological conditions, which means that they cannot be usefully employed to evaluate the functionality of the gastrointestinal tract, especially the colon, or alterations thereof. Finally, due to the possible uneven distribution of the contrast medium in the tract examined, false positives may be obtained.

Schering's EP 405 704 B1, EP 485 045 B1 and corresponding US patents cover new linear or cyclic polyaminopolycarboxylic acids derivatives which consent the visualization of both the renal and hepatobiliary systems. After intravenous administration, such agents are said to further consent the differentiation among the stomach, liver duodenum and pancreas. Cyclic contrast compounds, disclosed and claimed in US 5,871,709 and EP 485 045 B1, are nevertheless said to be particularly suitable for enteral and parenteral administration, and to consent an improved imaging, in particular of the liver.

US 6,039,931 includes examples in which linear complex compounds are administered intravenously in rats consenting a contrast increase in stomach, urinary bladder and intestinal loops as well as a marked enhancement of the liver.

US 4,999,445, WO 93/15093 and US 5,382,421 relate to new desferrioxamine B derivatives and a method using them as MRI contrast agents. When intravenously administered these derivatives are said to provide diagnostic information concerning the hepatobiliary system and the alimentary tract, especially the small intestine.

Claimed desferrioxamine B derivatives concentrate in bile and appear in the small intestine usually within about 2 minutes post injection.

Above cited patents in particular teach that claimed desferrioxamine B derivatives are the only complex compounds which can successfully be used when MRI imaging of the small intestine is desired rapidly after intravenous administration. Conventional hepatobiliary agents (such as Fe-EHPG and Fe-HBED), in fact, are said to show their appearance in the bile not before approximately 20 minutes post injection and furthermore, are said to only seldom produce the desired enhancement of the lumen of the small intestine when intravenously administered.

WO93/15093 discloses carboxylic acid derivatives of the iron (III) chelate of desferrioxamine B which are useful as magnetic resonance imaging contrast agents for renal, hepatobiliary and, in particular, small intestine image enhancement. When administered intravenously, these derivatives rapidly provide diagnostic information, usually within two minutes post injection.

WO93/03351 discloses amino acid-like magnetic resonance imaging contrast agents and a method of preparation thereof. The chelated complexes produce T1 contrast effect in the heart, liver, biliary tree and upper small intestine.

US5401493 discloses organic compounds for diagnostic imaging which contain at least one aryl group which has been derivatized to contain at least one perfluoro-1H, 1H-neopentyl moiety. Upon intravenous injection, these compounds allow fluorine MR imaging of the liver and upper intestinal lumen.

WO93/10821 discloses formulations useful for diagnostic imaging by nuclear magnetic resonance of the gastrointestinal tract, the bladder and the cavities of the female reproductive apparatus with the use f T2-weighted sequences.

It has now been unexpectedly discovered that MRI contrast agents which possess even a minimal level of hepatobiliary excretion, when administered intravenously, produce a considerable increase in the intensity of the MRI signal recorded in the intestine, and in particular in the colon.

This surprising result is obtained with no need for simultaneous administration of any other contrast agent, and above all without any major preparation of the patient. This means that very clear, well contrasted images of the intestinal lumen, and the colon in particular, can be obtained under basically physiological conditions, in a practically non-invasive way.

The result is even more surprising in view of the fact that these agents provide a strong, lasting increase in intraluminal contrast even when administered at the low doses commonly used in hepatobiliary imaging, for example.

This invention therefore relates to the use of contrast agents excreted by the hepatobiliary route, even in a partial or limited percentage but in any event equal to or exceeding 0.5% of the dose administered for the preparation of intravenous diagnostic contrast compositions for the virtual three-dimensional endoscopic visualisation of the colon by magnetic resonance imaging (M.R.I).

The compounds preferred for the use of the invention include, for example, paramagnetic chelates, which are suitable (*inter alia*) for imaging of the hepatobiliary system. Particularly preferred among them are the compounds commonly known as Gd-BOPTA and Gd-EOB-DTPA and their physiologically compatible salts, e.g. meglumine salts (MultiHance™) for the former and sodium salts (Eovist™) for the latter.

In particular, Gd-BOPTA in the form of the meglumine salt is an MRI contrast agent already marketed for liver imaging, which is excreted by both the renal and the biliary routes, although the latter only accounts for a very low proportion in man, namely around 2-4%. This contrast agent is soluble in water, and is commonly administered to patients intravenously. Preliminary results relating to its use in angiographic investigations conducted by magnetic resonance imaging have also been reported. Finally, pharmaceutical diagnostic compositions including the same agent for magnetic resonance imaging of the gastrointestinal tract have been described (patent applications WO 93/10821 and WO 98/28258) in which the said formulations are traditionally administered by the oral or aboral route, preferably after preparation of the patient.

As described below in the experimental section, it has now been quite unexpectedly discovered that despite its limited biliary excretion, Gd-BOPTA, after intravenous administration, generates a substantial increase in intraluminal contrast, and in particular in the contrast recorded in the colon, delineating its morphology in an extremely homogenous, clear, and complete way.

The concentration of the formulations administered is the same as normally used for liver imaging; however, the concentration can be modulated according to the part of the gastrointestinal tract to be examined. Generally such formulations have a concentration of contrast agent ranging from 0.001 to 1.0 mmol/mL, preferably from 0.01 to 0.5, more preferably from 0.01 to 0.25.

This new use of contrast media possessing at least partial biliary excretion offers great advantages.

The first is the possibility of displaying the gastrointestinal lumen under physiological conditions without subjecting the patient to uncomfortable and sometimes painful preparation.

The intensity and sharpness of the intraluminal contrast obtained with the new use of contrast agents in accordance with the invention and the great homogeneity of such contrast also makes it possible to display the real internal morphological situation of the intestine, clearly delineating any lesions, modifications and/or alterations of a structural nature, even in the presence of pathological manifestations of various kinds.

Such disorders, listed here by way of example but not of limitation, may be inflammatory diseases, irritable colon syndrome, peritonitis, constipation, polyposis, diverticulitis, perforations, cancerous forms, colorectal cancer, inflammation due to pharmacological treatment, such as long-term antibiotic treatment, or to chemotherapy, and so on.

Finally, post-surgical monitoring of the morphology of the gastrointestinal tract is possible.

As the diagnostic procedure is conducted under physiological conditions, an even more important aspect of the use of the contrast agents in accordance with the invention is the possibility of mapping the functional characteristics of the intestinal tract, thus allowing the radiologist to establish any functional alterations and/or abnormalities thereof of a pathological nature, or functional changes induced, for example, by pharmacological treatments, chemotherapy or surgery.

A further consequence of the possibility of conducting imaging under physiological conditions is the possible advantageous use of the agents in accordance with the invention to investigate biliary excretion disorders and study bile transport kinetics.

The fact that a high intraluminal signal intensity is obtained on average with the diagnostic doses in current use for hepatobiliary imaging, for example, means that the agents in accordance with the invention can be used for successive and combined visualisation of the liver, bile ducts, gall bladder and intestinal lumen after a single administration of the said agents. The use of the agents in accordance with the invention for joint visualisation of the gall bladder and intestinal lumen is particularly advantageous, and their use for joint visualisation of the gall bladder and colon is even more advantageous.

Finally, this new use of contrast agents with at least partial biliary excretion produces an increase in the intraluminal signal of such homogeneity and intensity as to make virtual three-dimensional visualisation of the lumen possible under physiological conditions; the said new use, and all the others described above, all constitute different aspects of the invention.

The MRI diagnostic technique is particularly preferred for this new use of contrast media in accordance with the invention in the first instance. However, other diagnostic techniques such as scintigraphy and X-ray radiography could conveniently be employed if combined with intravenous administration of suitable contrast agents possessing at least limited hepatobiliary excretion.

Of all the possible procedures for acquiring images, the use of three-dimensional angiographic sequences is preferred.

The response time varies from patient to patient, depending on the intestinal transit rate and also on the type of diet: an intense increase in intraluminal contrast in the colon is generally observed within 24 hours after administration. On average the intensity of the signal peaks between 10 and 70 hours, and preferably between 15 and 50 hours after injection of the contrast medium. However, in some patients an intense signal has been observed as long as 100 hours after administration, while partial signal intensity can be observed for up to 8 days after administration.

### Experimental example

Description of method of acquisition of MRI images of the luminal part of the colon using the three-dimensional angiography technique in healthy volunteers after intravenous injection of MultiHance™ (Gd-BOPTA meglumine salt).

A three-dimensional angiographic imaging technique was used because it provides advantageous visualisation of large regions of the anatomy, especially when associated with administration of contrast agents which are particularly effective in reducing the T1 relaxation times in standard MRI investigations.

In the trial conducted, the increased contrast in the hepatobiliary system and the gastrointestinal system was obtained with this kind of diagnostic technique, which produced up to 42 images of a three-dimensional volume measuring 40 x 32 x 12 cm in a single breath-hold.

The trial was conducted in six healthy volunteers aged between 22 and 29 years. The contrast agent used was Gd-BOPTA meglumine salt (MultiHance™), administered intravenously at a dose of 0.1 mmol/kg of body weight. The successive recording of images of the abdomen was performed 1, 12, 24, 36, 48, 70 and 105 hours after administration of the agent using the following 3D angio-sequence: 3D FLASH; TR 4.6 ms; TE 1.8 ms; α 50°C; rect. FOV 390 mm (6/8); Ma: 215 x 512; acq 28 s; slab thickness 120 mm; 42 sections. Three volunteers were re-tested 14 days after administration of the agent.

T1-weighted axial images of the liver and abdomen were recorded in addition to the 3D angio-sequences referred to above.

The visualisation procedure was conducted with an MRI scanner operating at 1.5 Tesla (Magneton Vision Plus, Siemens Medical Systems, Erlangen, Germany) equipped with a phased array body coil.

The images were processed with standard MIP software on a dedicated 3D MR workstation (Virtuoso, Siemens Medical System).

No preparation, medication or joint administration of other drugs or other contrast media was performed on the volunteers studied.

An intense increase in intraluminal contrast was recorded in all volunteers within a maximum of 24 hours after administration of MultiHance™. The faeces presented a homogenous contrast in the lumen, thus indicating optimum mixing with the contrast agent. This homogenous increase in the signal was so intense as to allow virtual three-dimensional endoscopy. The optimum post-injection time interval for obtaining the required images proved to be between 15 and 50 hours. In some patients an intense intraluminal signal was detected in the colon as much as 100 hours after administration, while a partial increase was still detectable after 8 days.

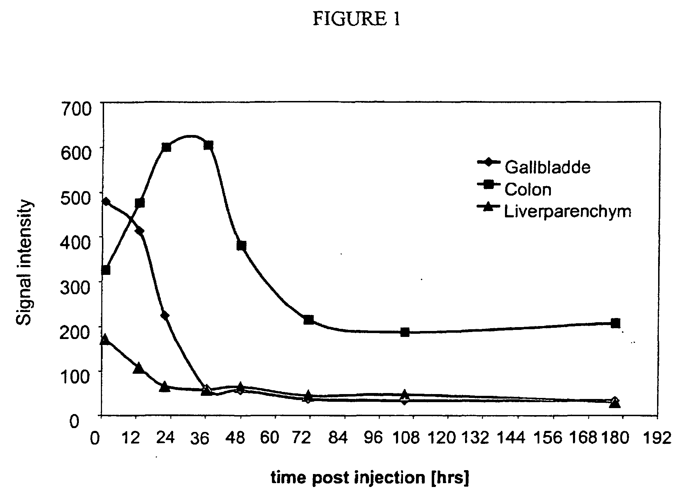

Before performing colonography, it was possible to investigate liver function by recording the signal intensity in the liver parenchyma and gall bladder in the first few hours after administration of the contrast agent. The increased signal intensity recorded in the these organs is illustrated in the graph in Figure 1, which shows the intensity of the signal in relation to time in the colon, liver parenchyma and gall bladder as the average of the results obtained in the tests on the six healthy volunteers. The fastest reduction in signal intensity was observed in the liver parenchyma, the signal being halved within 10 hours of administration. The signal intensity recorded in the gall bladder was more intense and persistent, with a half-life of 15 hours. The greatest signal intensity was observed in the colon between 15 and 40 hours after administration. This seems to be the ideal period for recording three-dimensional MRI colonography.

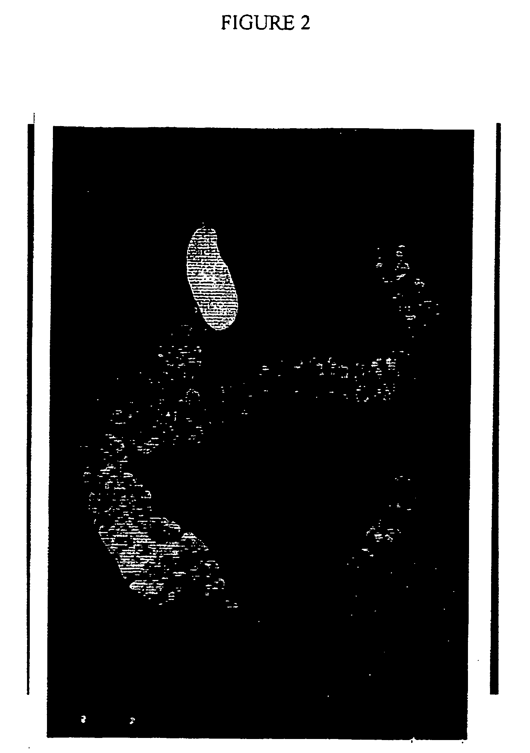

Figure 2 shows 3D MR colonography registered 24 hours after injection of 0.1 mmol/kg BW gadobenate dimeglumine. No bowel preparation was performed and no medication was given before imaging. We can note the still intense enhancement within the gallbladder. Homogenous signal can be seen in all segments of the colon within the field of view. It results very clear the delineation of the colonic haustration. The images were acquired within 28 s and are displayed as a maximum intensity projection. Since the data are acquired as a 3D set, real time fly through and visualisation in any direction is possible.

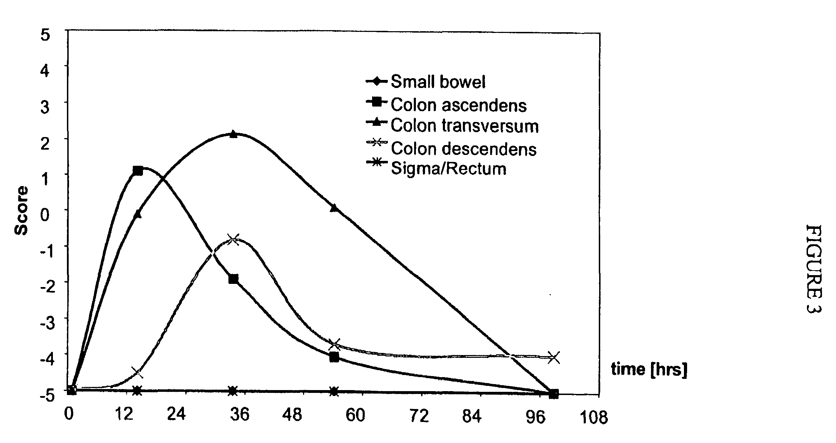

Figure 3 shows the median qualitative assessment score of a blinded reader for the diagnostic visualisation in respect to time and anatomic location.

## Claims

1. Use of contrast agents excreted by the hepatobiliary route in a percentage equal to or exceeding 0.5% of the dose administered for the preparation of intravenous diagnostic contrast compositions for the virtual three-dimensional endoscopic visualisation of the colon by magnetic resonance imaging (MRI).

2. Use of contrast agents according to claim 1, wherein said visualisation is obtained under physiological conditions.

3. Use of contrast agents according to claim 1, for the visualisation of the morphology of the colon.

4. Use of contrast agents according to claim 1, for the visualisation of morphological/structural or pathological lesions, modifications and/or alterations of the colon.

5. Use of contrast agents according to claim 1, for the visualisation of functional abnormalities.

6. Use of contrast agents according to claims 4 and 5, where said functional lesions, modifications, alterations and/or abnormalities are caused by inflammatory states, peritonitis, irritable colon syndrome, constipation, polyposis, diverticulitis, cancerous disease, perforations and/or pharmacological treatments.

7. Use of contrast agents according to claim 1, for the visualisation of the colon after surgery.

8. Use of contrast agents according to claim 1 for the combined and sequential visualisation of the liver, bile ducts, gall bladder and colon with a single administration of said agents.

9. Use of contrast agents according to claim 1, in which said visualisation is obtained with three-dimensional angiographic sequences.

10. Use of contrast agents according to any preceding claims, in which the patient is not subjected to any kind of preparation or pre-treatment prior to administration of the contrast medium.

11. Use of contrast agents according to claim 1, in which said agents are Gd-BOPTA, Gd-EOB-DTPA and/or their physiologically compatible salts.

12. Use of contrast agents according to claim 1 for the preparation of diagnostic compositions suitable for use in studies of bile transport kinetics.

## Revendications

1. Utilisation d'agents de contraste excrétés par la voie hépatobiliaire dans un pourcentage égal à ou dépassant 0,5 % de la dose administrée, pour la préparation de compositions de contraste de diagnostiques intraveineuses permettant la visualisation endoscopique tridimensionnelle virtuelle du côlon par imagerie par résonance magnétique (IRM).

2. Utilisation d'agents de contraste selon la revendication 1, dans laquelle ladite visualisation est obtenue dans des conditions physiologiques.

3. Utilisation d'agents de contraste selon la revendication 1, pour la visualisation de la morphologie du côlon.

4. Utilisation d'agents de contraste selon la revendication 1, pour la visualisation de lésions, modifications et/ou altérations morphologiques/ structurelles ou pathologiques du côlon.

5. Utilisation d'agents de contraste selon la revendication 1, pour la visualisation d'anomalies fonctionnelles.

6. Utilisation d'agents de contraste selon les revendications 4 et 5, où lesdites lésions, modifications, altérations et/ou anomalies fonctionnelles sont causées par des états inflammatoires, une péritonite, un syndrome du côlon irritable, une constipation, une polypose colique, une diverticulite, une maladie cancéreuse, des perforations et/ou des traitements pharmacologiques.

7. Utilisation d'agents de contraste selon la revendication 1, pour la visualisation du côlon après une intervention chirurgicale.

8. Utilisation d'agents de contraste selon la revendication 1, pour la visualisation combinée et séquentielle du foie, des canaux cholédoques, de la vésicule biliaire et du côlon, avec une administration unique desdits agents.

9. Utilisation d'agents de contraste selon la revendication 1, dans laquelle ladite visualisation est obtenue avec des séquences angiographiques tridimensionnelles.

10. Utilisation d'agents de contraste selon l'une quelconque des revendications précédentes, dans laquelle le patient n'est soumis à aucune sorte de préparation ou de prétraitement avant l'administration du milieu de contraste.

11. Utilisation d'agents de contraste selon la revendication 1, dans laquelle lesdits agents sont le Gd-BOPTA, le Gd-EOB-DTPA et/ou leurs sels physiologiquement compatibles.

12. Utilisation d'agents de contraste selon la revendication 1, pour la préparation de compositions diagnostiques, appropriées pour être utilisées dans des études de cinétique de transport biliaire.

## Patentansprüche

1. Verwendung von Kontrastmitteln, welche in einer Menge von gleich oder mehr als 0,5% der verabreichten Dosis auf dem hepatobiliären Weg ausgeschieden werden, zur Herstellung von intravenösen diagnostischen Kontrastzusammensetzungen für die virtuelle dreidimensionale endoskopische Bildgebung des Kolons durch Magnetresonanz-Bildgebung (MRI).

2. Verwendung von Kontrastmitteln gemäß Anspruch 1, wobei die Bildgebung unter physiologischen Bedingungen erhalten wird.

3. Verwendung von Kontrastmitteln gemäß Anspruch 1 zur Bildgebung der Morphologie des Kolons.

4. Verwendung von Kontrastmitteln nach Anspruch 1 zur Bildgebung von morphologischen/strukturellen oder pathologischen Läsionen, Modifikationen und/oder Veränderungen des Kolons.

5. Verwendung von Kontrastmitteln gemäß Anspruch 1 zur Bildgebung funktioneller Abnormitäten.

6. Verwendung von Kontrastmitteln gemäß der Ansprüche 4 und 5, wobei die funktionellen Läsionen, Modifikationen, Änderungen und/oder Abnormitäten durch entzündliche Zustände, Peritonitis, Reizdarm-Syndrom, Verstopfungen, Polyposis, Divertikulitis, Krebserkrankungen, Perforationen und/oder pharmakologische Behandlungen hervorgerufen werden.

7. Verwendung von Kontrastmitteln gemäß Anspruch 1 zur Bildgebung des Kolons nach Operationen.

8. Verwendung von Kontrastmitteln gemäß Anspruch 1 zur kombinierten und sequentiellen Bildgebung der Leber, der Gallengänge, der Gallenblase und des Kolons mit einer einzigen Verabreichung der Mittel.

9. Verwendung von Kontrastmitteln gemäß Anspruch 1, wobei die Bildgebung durch dreidimensional angiographische Sequenzen erhalten wird.

10. Verwendung von Kontrastmitteln gemäß einem der vorangegangenen Ansprüche, bei welcher der Patient vor der Verabreichung des Kontrastmittels keinerlei Vorbereitung oder Vorbehandlung unterzogen wird.

11. Verwendung von Kontrastmitteln gemäß Anspruch 1, wobei die Mittel Gd-BOPTA, Gd-EOB-DTPA und/oder deren physiologisch verträgliche Salze sind.

12. Verwendung von Kontrastmitteln gemäß Anspruch 1 zur Herstellung diagnostischer Zusammensetzungen, welche für die Verwendung in Studien von Gallen-Transport-Kinetiken geeignet sind.
